# EUROPEAN PATENT SPECIFICATION

(11) **EP 2 617 807 B1**
(45) Date of publication and mention of the grant of the patent: **21.10.2020**
(21) Application number: 11825017.4
(22) Date of filing: 05.09.2011
(51) Int. Cl.: C12M 3/00

(54) **CULTURE SUBSTRATE**
KULTURSUBSTRAT
SUBSTRAT DE CULTURE

(30) Priority: 14.09.2010 JP 2010205305
(43) Date of publication of application: 24.07.2013
(73) Proprietor: AGC TECHNO GLASS CO., LTD., Shizuoka 421-0302 (JP)
(72) Inventor: ITOH, Tohru, Funabashi-shi Chiba 273-0044 (JP); TADA, Yutaka, Funabashi-shi Chiba 273-0044 (JP); WADA, Aya, Funabashi-shi Chiba 273-0044 (JP)
(74) Representative: Müller-Boré & Partner Patentanwälte PartG mbB
(86) International application number: PCT/JP2011/070170
(87) International publication number: WO 2012/036011

(56) References cited:
- WO-A1-2008/156041
- JP-A- 2001 522 242
- JP-A- 2003 503 021
- JP-A- 2009 050 194
- JP-A- 2010 088 347
- US-A- 6 027 695
- US-A1- 2010 068 793

## Description

### TECHNICAL FIELD

The present invention relates to a culture substrate to culture objects to be cultured such as cells and tissues thereby to prepare spheroids.

### BACKGROUND ART

In recent years, instead of monolayer culturing to two-dimensionally culture cells, spheroid culturing to culture cells and three-dimensionally aggregate them has attracted attention. By the spheroid culturing, cells in a state closer to cells in the body can be constituted as compared with the monolayer culturing, and specific functions which cells have in the body can be brought out.

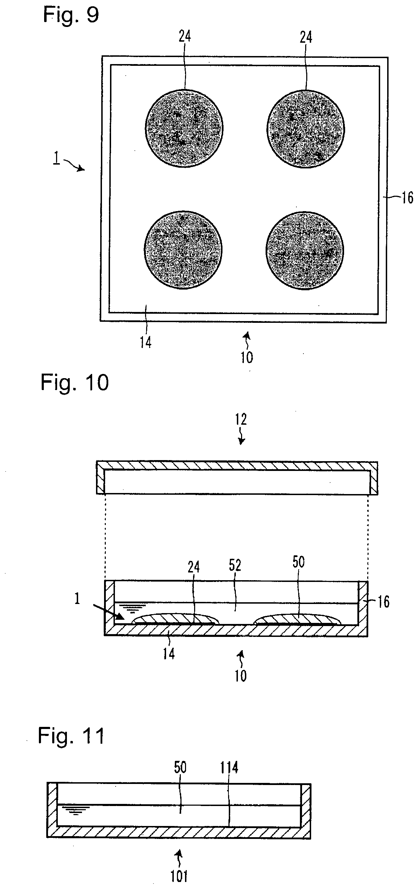

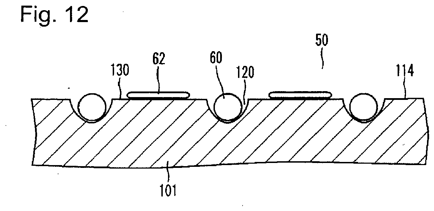

As one example of a conventional culture substrate 101 to carry out the spheroid culturing, for example, a container as shown in Fig. 11 may be mentioned. At the bottom 114 of this container, a plurality of dents 120 are formed with intervals therebetween, as shown in Fig. 12. The bottom 114 of the container is coated with a cell adhesion inhibitor (not shown) (Patent Document 1).

By the spheroid culturing, a culture fluid 50 in which cells as spheroid precursors are stirred is poured into the culture container, and the cells are cultured in the dents 120. The cells in the dents 120 are cultured in accordance with the shape and the size of the dents 120, are aggregated three-dimensionally and form spheroids 60 as shown in Fig. 12.

US 6027695 A describes a microtiter plate comprising a plurality of wells having concave bottom surfaces and being separated by walls having non-flat upper surfaces.

### PRIOR ART DOCUMENT

### PATENT DOCUMENT

Patent Document 1: WO2007/055056

### DISCLOSURE OF INVENTION

### TECHNICAL PROBLEM

However, if spheroid culturing is carried out by using the above-described conventional culture substrate 101, there have been such problems that cells 62 cultured in monolayer and spheroids with non-uniform sizes are formed in a large amount in addition to the spheroids 60. The reason is considered to be such that in the conventional culture substrate 101, as shown in Fig. 12, flat surfaces 130 are formed on the substrate surface between adjacent dents 120. In a case where the flat surfaces 130 are formed like this case, if a culture fluid 50 in which cells are stirred is poured into the culture container, cells are precipitated even on the flat surfaces 130. If such cells are present in the culture container, in addition to spheroids 60 in accordance with the size of the dents, cells may be two-dimensionally cultured, or spheroids in random sizes which are unaffected by the size of the dents may form. As mentioned above, with the conventional culture substrate 101, culturing to uniformly form spheroids having a desired size cannot efficiently be carried out. These cells cultured in monolayer or spheroids in random sizes may have different physiological functions, or in a case of stem cell culturing, may be in a different stage of differentiation as compared with uniform spheroids in accordance with the size of the dents. Thus, the uniformity of the cell groups in the culture container tends to be low, thus impairing the evaluation of experimental data, etc.

Under these circumstances, the present invention has been made to solve the above problems, and its object is to provide a culture substrate with which spheroids having a desired size can be uniformly formed, and culturing can efficiently be carried out.

### SOLUTION TO PROBLEM

To achieve the above object, the culture substrate of the present invention is characterized by having a plurality of dents to form compartments in which objects to be cultured are cultured, on the culture substrate surface, the culture substrate surface between adjacent dents being a non-flat surface.

### ADVANTAGEOUS EFFECTS OF INVENTION

According to the present invention, spheroid culturing can efficiently be carried out.

### BRIEF DESCRIPTION OF DRAWINGS

Fig. 1 is an oblique view illustrating a culture substrate according to a first embodiment of the present invention.
Fig. 2 is a vertical cross sectional view illustrating a culture substrate according to a first embodiment of the present invention.
Fig. 3 is a partial oblique view illustrating a well-forming region of a culture substrate according to a first embodiment of the present invention.
Fig. 4 is a cross sectional view at the arrow IV-IV of Fig. 3.
Fig. 5 is a cross sectional view at the arrow V-V of Fig. 3.
Fig. 6 is a plan view schematically illustrating irradiation spots with laser light on the surface of a culture substrate according to a first embodiment of the present invention.
Fig. 7 is a partial cross sectional view illustrating a well-forming region of a culture substrate according to a second embodiment of the present invention.
Fig. 8 is a vertical cross sectional view illustrating a state where a culture substrate according to a third embodiment of the present invention is placed in a petri dish.
Fig. 9 is a plan view illustrating a culture substrate according to a fourth embodiment of the present invention.
Fig. 10 is a vertical cross sectional view illustrating a culture substrate according to a fourth embodiment of the present invention.
Fig. 11 is a cross sectional view illustrating a conventional culture substrate.
Fig. 12 is a cross sectional view schematically illustrating objects to be cultured on the surface of a conventional culture substrate.

### DESCRIPTION OF EMBODIMENTS

### [First embodiment]

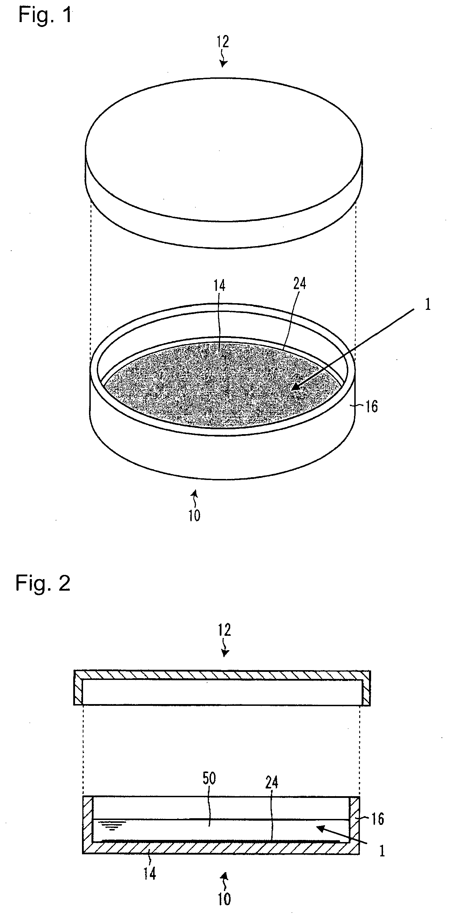

A culture substrate according to a first embodiment of the present invention will be described with reference to Figs. 1 to 6.

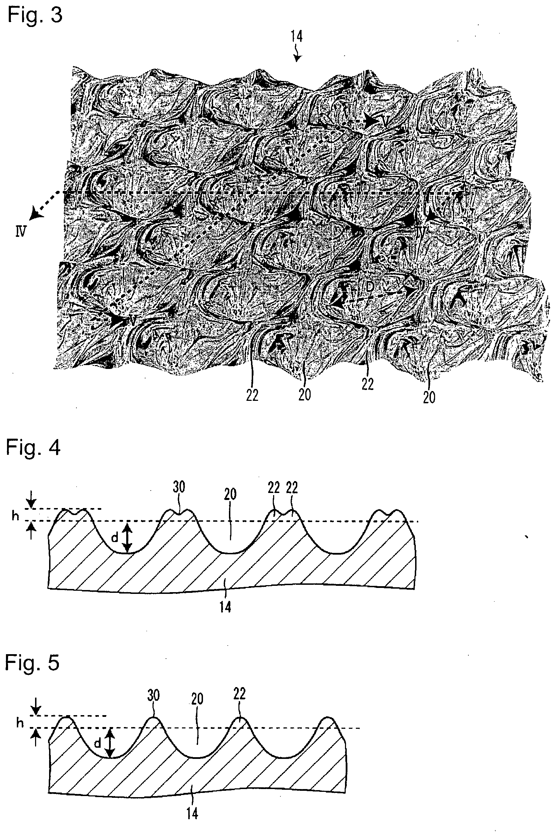

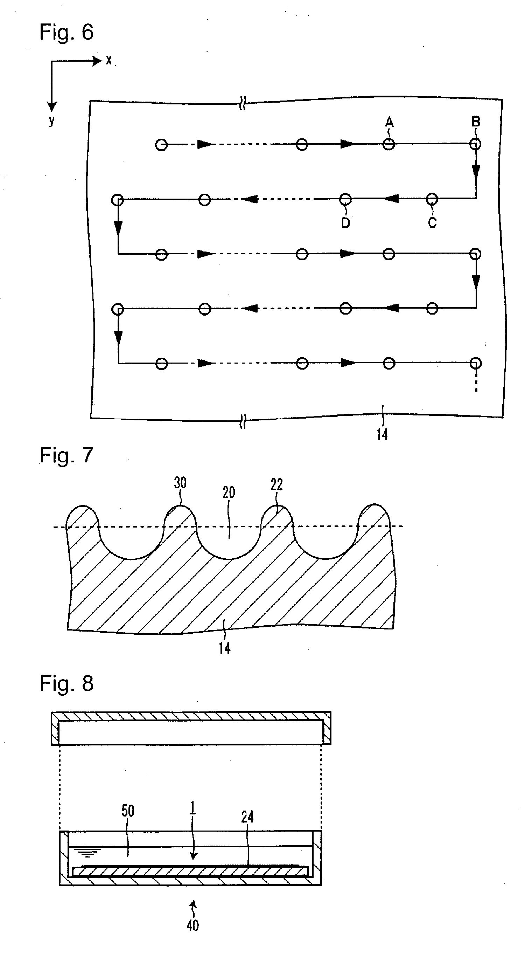

Fig. 1 is an oblique view illustrating a culture container having a culture substrate of the present invention. Fig. 2 is a vertical cross sectional view illustrating a culture container. Fig. 3 is a partial oblique view illustrating a well-forming region of a culture substrate. Fig. 4 is a cross sectional view at the arrow IV-IV of Fig. 3. Fig. 5 is a cross sectional view at the arrow V-V of Fig. 3. Fig. 6 is a plan view illustrating irradiation spots with laser light applied to the surface of a culture substrate.

The culture substrate 1 according to this embodiment constitutes a main portion of a culture container to prepare spheroids (cell aggregates) by culturing cells and three-dimensionally aggregating them.

First, the constitution of the culture substrate 1 according to this embodiment will be described.

A culture container has a container main body 10 and a lid 12 as shown in Figs. 1 and 2. In this example, a baseplate portion 14 in the inside of the container main body 10 is a portion corresponding to the culture substrate 1. The baseplate portion 14 in the inside of the container main body 10, i.e. the culture substrate 1, is made of a synthetic resin, for example, polystyrene. In this embodiment, the culture substrate 1 is obtained by injection molding using a synthetic resin material.

The container main body 10 has a disk-shape baseplate portion 14 and a cyclic side wall portion 16. The side wall portion 16 stands up from the periphery of the baseplate portion 14. In this embodiment, the baseplate portion 14 is designed to have a diameter of 85 mm and a thickness of 1 mm. Further, the side wall portion 16 is designed to have a height of 20 mm.

The lid 12 is formed into a shape corresponding to an opening of the container main body 10. The lid 12 is used to cover the container main body 10 so as to maintain the cell culture environment.

On a well-forming region 24 (that is, a region on which compartments in which objects to be cultured are cultured are formed) on the upper surface of the baseplate portion 14 (that is, the upper surface of the culture substrate corresponding to the surface in the inside of the container main body 10), a plurality of dents 20 are formed as shown in Figs. 2 to 5. The inner surface of each dent 20 is a smooth concave plane. Each dent 20 forms a compartment (well) in which objects to be cultured are cultured. In this embodiment, on a circular well-forming region 24 having a diameter of 85 mm, about 14,200 dents (about 250 dents/cm²) 20 are formed.

In this embodiment, the dents 20 are formed by irradiating the well-forming region 24 on the culture substrate surface with laser light. The laser irradiation is carried out by irradiating the upper surface of the baseplate portion 14 placed in an x-y plane with laser light in a z axis direction as shown in Fig. 6.

First, while an irradiation portion of a laser irradiation apparatus is moved in a position direction of the x axis, laser light is applied at certain intervals (for example, 800 µm) to form an array of a plurality of dents 20 in the x axis direction. Then, the irradiation portion is moved in the y axis direction for a certain distance (for example, 400 µm), and then while the irradiation portion is moved in a negative direction of the x axis, laser light is applied at certain intervals (for example 800 µm) to form an array of a plurality of dents 20 in the x axis direction. In the same manner, the irradiation portion is moved in the y axis direction for a certain distance (for example, 400 µm). These operations are repeatedly carried out to form a plurality of dents regularly aligned on the upper surface of the baseplate portion 14.

In this embodiment, as shown in Fig. 6, assuming that the central coordinate (x, y) of the irradiation spot A is the origin (0, 0), the center of the irradiation spot B adjacent to the irradiation spot A is located at (0.8, 0), the center of the irradiation spot C is located at (0.4, 0.4), and the center of the irradiation spot D is located at (-0.4, 0.4). As the x coordinates of the irradiation spots A and B and the x coordinates of the irradiation spots C and D are shifted, a plurality of dents 20 can be densely formed on the well-forming region 24. The dents 20 are formed preferably in a density of from 10 dents/cm² to 10,000 dents/cm² per unit area of the well-forming region 24 of the culture substrate 1, more preferably from 20 dents/cm² to 8,000 dents/cm², further preferably from 20 dents/cm² to 3,000 dents/cm².

The above "from ··· to ···" indicating the range of values is meant to include the values as the lower limit and the upper limit, unless otherwise specified, and hereinafter in this specification, "from ··· to ···" is used to have the same meaning.

In this embodiment, a CO₂ laser is used as a laser light source, and the laser light is applied by pulse irradiation at an output power of 10 W at an irradiation rate of 6,100 mm/min. The shape of the irradiation spot is circular, and its diameter is about 400 µm. If the spheroids are too small, no desired physiological function will be obtained, and if they are too large, the central portions of the spheroids undergo necrosis. Considering these points, the diameter of the irradiation spot is appropriately from 20 to 1,500 µm.

Although the shape of the irradiation spot is circular, the shape of the opening of each dent 20 is flattened into a substantially elliptical shape. This flatness of the opening shape is considered to be attributable to the direction in which the synthetic resin material is poured into a mold at the time of molding the container main body 10.

By application of laser light to the culture substrate surface (the upper surface of the baseplate portion 14), the synthetic resin material constituting the baseplate portion 14 is melted, whereby dents 20 are formed. Further, at the peripheries of the openings of the dents 20, the molten synthetic resin material is piled up to form banks 22 as shown in Figs. 3 to 5.

In this embodiment, the two adjacent dents 20 are formed via one or two banks 22, and on the culture substrate surface between adjacent dents 20, no flat surface remains. That is, the culture substrate surface between adjacent dents 20 forms a non-flat surface 30. In the cross sectional view at the arrow IV-IV of Fig. 3 shown in Fig. 4, two banks 22 present between two adjacent dents 20 are connected to each other to form the non-flat surface 30.

The distance between adjacent dents 20, the diameter and the depth of each dent 20, the width and the height of each bank 22 and the like can be adjusted by adjusting the irradiation conditions such as the laser light irradiation position and the output power. In this embodiment, laser irradiation is carried out by setting the laser light irradiation conditions so that no flat surface remains on the culture substrate surface between adjacent dents 20, that is, the culture substrate surface between adjacent dents 20 is the non-flat surface 30.

The depth (i.e. the depth based on the upper surface of the baseplate portion 14 (i.e. the culture substrate) before laser irradiation, as shown in Figs. 4 and 5) d of each dent 20 is preferably designed to be from 10 to 1,500 µm, and in this embodiment, it is designed to be 200 ± 20 µm. Further, the thickness of the baseplate portion 14 is properly designed depending upon the depth d so that the baseplate portion 14 will not have a hole. Further, the major axis (the major axis on the upper surface of the baseplate portion 14 before laser irradiation) D of the opening of each substantially elliptic dent 20 is preferably designed to be from 10 to 1,500 µm, and in this embodiment, it is designed to be 500 ± 20 µm. Further, the height (i.e. the height based on the upper surface of the baseplate portion 14 before laser irradiation as shown in Figs. 4 and 5) h of each bank 22 is preferably designed to be from 10 to 50 µm, and in this embodiment, it is designed to be 25 ± 5 µm.

The upper surface of the baseplate portion 14, i.e. the surface of the portion corresponding to the culture substrate, is preferably coated with a cell adhesion inhibitor (not shown). The culture substrate cell adhesion inhibitor has a role to inhibit cells from adhering to the upper surface of the baseplate portion 14, particularly the inner surfaces of the dents 20. As the cell adhesion inhibitor, for example, a phospholipid polymer, polyhydroxyethyl methacrylate or polyethylene glycol may, for example, be used.

Now, a method of culturing objects to be cultured using the culture substrate 1 according to this embodiment will be described.

Cells as spheroid precursors which are objects to be cultured are put in a culture fluid 50 and stirred. After stirring, the culture fluid 50 is poured into a container main body 10 (see Fig. 2), whereupon the cells in the culture fluid 50 precipitate and fit into dents 20.

Then, the container main body 10 is covered with a lid 12 and is left to stand for several days to several tens days. The cells in the dents 20 are cultured and grow. On that occasion, as the inner surfaces of the dents 20 are coated with the cell adhesion inhibitor, the cells three-dimensionally aggregate in accordance with the shape and the size of the dents 20. In such a manner, spheroids are obtained.

Now, the effects of the culture substrate 1 according to this embodiment will be described.

According to this embodiment, the culture substrate surface between adjacent dents 20 is the non-flat surface 30. Accordingly, the precipitating objects to be cultured are likely to fit into the dents 20.

In a conventional culture substrate 101, flat surfaces 130 are formed on the culture substrate surface between adjacent dents 120. Accordingly, on the flat surfaces 130, cells may be cultured in monolayer, or spheroids in random sizes which are unaffected by the size of the dents 120 may form. On the other hand, in the culture substrate 1 according to this embodiment, the culture substrate surface between adjacent dents 20 forms a non-flat surface 30. Accordingly, cells are less likely to be cultured in monolayer, or non-uniform spheroids are less likely to form, the probability of uniform spheroids forming is high, and the spheroid culturing can efficiently be carried out.

The size of the spheroids to be prepared varies depending upon the purpose of use of the spheroids, the type of cells to be cultured, and the like. Accordingly, to prepare spheroids, it is necessary to prepare a culture substrate 1 having dents 20 in accordance with the desired size of spheroids. Here, in this embodiment, the dents 20 and the banks 22 are formed by laser irradiation. Accordingly, by adjusting the irradiation conditions such as the irradiation position and the output power, it is possible to easily form dents 20 and banks 22 having optional sizes on the culture substrate 1.

Further, in a case where a transparent synthetic resin material such as polystyrene is used as the culture substrate, and dents are formed by laser light irradiation on the synthetic resin material, the dents 20 open upward in their cross section, and the inner surfaces of the dents 20 are smooth by heat of the laser light, thus reducing diffuse reflection of transmitted light, whereby the spheroids to be cultured in the dents 20 can easily be observed by a microscope.

### [Second embodiment]

A culture substrate according to a first embodiment of the present invention will be described with reference to Fig. 7. Fig. 7 is a partial cross sectional view illustrating a well-forming region of a culture substrate. This embodiment is a modified example of the first embodiment, and for the same components and the analogous components in the first embodiment, the same symbols are used, and duplicated explanation will be omitted.

In the first embodiment, a plurality of dents 20 and banks 22 are formed by irradiating the culture substrate surface with laser light. On the other hand, in this embodiment, the culture substrate 1 is formed by injection molding a synthetic resin material using a mold having convexes to form a plurality of dents 20 and concaves to form banks 22. The plurality of dents 20 and banks 22 are formed simultaneously with molding of the culture substrate 1.

In this embodiment, as shown in Fig. 7, on the culture substrate surface of the culture substrate 1, hemispherical dents 20 and banks 22 which are semi-circular in their cross section are formed. By producing the culture substrate 1 by injection molding using a mold, more highly uniform dents 20 can be formed, and uniformity of spheroids to be formed can be made high.

### [Third embodiment]

A culture substrate according to a first embodiment of the present invention will be described with reference to Fig. 8. Fig. 8 is a vertical cross sectional view illustrating a state where a culture substrate is placed in a petri dish. This embodiment is a modified example of the first embodiment, and for the same components and the analogous components in the first embodiment, the same symbols are used, and duplicated explanation will be omitted.

In the culture substrate 1 according to the first embodiment, a plurality of dents 20 are formed on the bottom (the upper surface of the baseplate portion 14) in the inside of the culture container. On the other hand, a culture substrate 1 according to this embodiment comprises a synthetic resin material formed into a disk shape. The culture substrate 1 is formed in such a manner that a synthetic resin material is injection molded into a disk-shape substrate, and one surface is irradiated with laser light to form a plurality of dents 20 on the one surface of the culture substrate 1.

The culture substrate 1 according to this embodiment is used as placed in a petri dish 40 made of glass for example, as shown in Fig. 8. According to this embodiment, formation of the culture substrate 1 is easy, and the production cost can be suppressed, as compared with the first embodiment.

### [Fourth embodiment]

A culture substrate according to a fourth embodiment of the present invention will be described with reference to Figs. 9 and 10. Fig. 9 is a plan view illustrating a culture substrate. Fig. 10 is a vertical cross sectional view illustrating a culture container having a container main body provided with a culture substrate and a lid. This embodiment is a modified example of the first embodiment, and for the same components and the analogous components in the first embodiment, the same symbols are used, and duplicated explanation will be omitted.

To a culture substrate 1 according to this embodiment, a baseplate portion 14 of a container main body 10 corresponds as shown in Fig. 9, and on the upper surface of the baseplate portion 14, there are four circular well-forming regions 24. The four well-forming regions 24 are disposed with distances therebetween. Further, in each of the well-forming regions 24 of the culture substrate 1, a plurality of dents to form compartments in which objects to be cultured are cultured are formed on the substrate surface, and the substrate surface between adjacent dents forms a non-flat surface.

In this embodiment, cells as spheroid precursors are put by using a pipet or the like, and a stirred culture fluid 50 or a culture fluid 50 containing fertilized eggs is dropped on the four well-forming regions 24. Then, as shown in Fig. 10, mineral oil 52 is poured into the container main body 10. On that occasion, the culture fluid 50 and the mineral oil 52 are not miscible. In such a state, the container is left to stand for several days to several tens days to prepare spheroids or to culture the fertilized eggs.

According to this embodiment, it is possible to prepare several types of spheroids by a single culture substrate 1.

### [Other embodiments]

The above respective embodiments are typical examples, and the present invention is not limited thereto. For example, the material of the culture substrate 1 may be glass, not a synthetic resin material. Further, the shape and the size of the culture substrate 1 may optionally be designed. Further, the shape and the size of the dents 20 and the banks 22 may also be optionally designed depending upon the shape and the size of cells to be cultured or desired spheroids.

Further, the above embodiments may be combined. For example, the dents 20 on the disk-shape culture substrate 1 according to the third embodiment may be formed by injection molding as described for the second embodiment.

### INDUSTRIAL APPLICABILITY

According to the present invention, since the culture substrate surface between adjacent dents is a non-flat surface, precipitating objects to be cultured are likely to fit into the dents, and since the culture substrate surface is the non-flat surface, two dimensional culturing in monolayer or formation of non-uniform spheroids is less likely to occur, the probability of three-dimensionally uniformly aggregated spheroids forming is high, and spheroid culturing can efficiently be carried out.

### REFERENCE SYMBOLS

1: Culture substrate, 10: container main body, 12: lid, 14: baseplate portion, 16: side wall portion, 20: dent, 22: bank, 24: well-forming region, 30: non-flat surface, 40: petri dish, 50: culture fluid, 52: mineral oil

## Claims

1. A culture substrate having a plurality of dents to form compartments in which objects to be cultured are cultured, on the culture substrate surface, the culture substrate surface between adjacent dents being a non-flat surface, wherein the non-flat surface present between adjacent dents has banks and the inner surface of each dent is a smooth concave plane.

2. The culture substrate according to Claim 1, wherein at least the inner surfaces of the dents are coated with a cell adhesion inhibitor.

3. The culture substrate according to Claim 1 or 2, wherein the diameter of the openings of the dents is at least 20 µm and at most 1,500 µm.

4. The culture substrate according to any one of Claims 1 to 3, wherein the depth of the dents is at least 10 µm and at most 1,500 µm.

5. The culture substrate according to any one of Claims 1 to 4, wherein the plurality of dents are formed on the surface in a well-forming region on the culture substrate surface in a density of from 10 dents/cm² to 10,000 dents/cm².

6. The culture substrate according to any one of Claims 1 to 5, wherein the dents are formed by laser irradiation on the culture substrate surface.

7. The culture substrate according to any one of Claims 1 to 6, which is made of a synthetic resin.

8. A culture container having the culture substrate as defined in any one of Claims 1 to 7.

## Patentansprüche

1. Kultursubstrat, das eine Vielzahl von Vertiefungen aufweist, um Kompartimente zu bilden, in denen zu kultivierende Objekte auf der Kultursubstratoberfläche kultiviert werden, wobei die Kultursubstratoberfläche zwischen benachbarten Vertiefungen eine nicht-flache Oberfläche ist, wobei die zwischen benachbarten Vertiefungen vorhandene nicht-flache Oberfläche Überhöhungen aufweist und die innere Oberfläche jeder Vertiefung eine glatte konkave Fläche ist.

2. Kultursubstrat nach Anspruch 1, wobei mindestens die inneren Oberflächen der Vertiefungen mit einem Zelladhäsionsinhibitor beschichtet sind.

3. Kultursubstrat nach Anspruch 1 oder 2, wobei der Durchmesser der Öffnungen der Vertiefungen mindestens 20 µm und höchstens 1.500 µm beträgt.

4. Kultursubstrat nach einem der Ansprüche 1 bis 3, wobei die Tiefe der Vertiefungen mindestens 10 µm und höchstens 1.500 µm beträgt.

5. Kultursubstrat nach einem der Ansprüche 1 bis 4, wobei die Vielzahl der Vertiefungen auf der Oberfläche in einem Wells bildenden Bereich auf der Kultursubstratoberfläche in einer Dichte von 10 Vertiefungen/cm² bis 10000 Vertiefungen/cm² gebildet werden.

6. Kultursubstrat nach einem der Ansprüche 1 bis 5, wobei die Vertiefungen durch Laserbestrahlung auf der Kultursubstratoberfläche gebildet werden.

7. Kultursubstrat nach einem der Ansprüche 1 bis 6, das aus einem synthetischen Harz hergestellt ist.

8. Kulturbehälter, der das Kultursubstrat wie in einem der Ansprüche 1 bis 7 definiert aufweist.

## Revendications

1. Substrat de culture comportant une pluralité de bosselures, destinées à former les compartiments dans lesquels des objets à cultiver sont cultivés, sur la surface de substrat de culture, la surface de substrat de culture entre des bosselures adjacentes étant une surface non plate, dans lequel la surface non plate présente entre des bosselures adjacentes comporte des talus et la surface interne de chaque bosselure est un plan concave lisse.

2. Substrat de culture selon la revendication 1, dans lequel au moins les surfaces internes des bosselures sont revêtues d'un inhibiteur d'adhérence cellulaire.

3. Substrat de culture selon la revendication 1 ou 2, dans lequel le diamètre des ouvertures des bosselures est supérieur ou égal à 20 µm et inférieur ou égal à 1500 µm.

4. Substrat de culture selon l'une quelconque des revendications 1 à 3, dans lequel la profondeur des bosselures est supérieure ou égale à 10 µm et inférieure ou égale à 1500 µm.

5. Substrat de culture selon l'une quelconque des revendications 1 à 4, dans lequel la pluralité de bosselures est formée sur la surface dans une zone de formation de puits sur la surface de substrat de culture avec une densité partant de 10 dents/cm² jusqu'à 10000 dents/cm².

6. Substrat de culture selon l'une quelconque des revendications 1 à 5, dans lequel les bosselures sont formées par rayonnement laser sur la surface de substrat de culture.

7. Substrat de culture selon l'une quelconque des revendications 1 à 6, qui est réalisé en une résine synthétique.

8. Conteneur de culture comportant le substrat de culture selon l'une quelconque des revendications 1 à 7.
